# EUROPEAN PATENT SPECIFICATION

(11) **EP 0 610 976 B1**
(45) Date of publication and mention of the grant of the patent: **07.05.1997**
(21) Application number: 94200047.2
(22) Date of filing: 11.01.1994
(51) Int. Cl.: A61F 5/48

(54) **Moisture absorbing structure**
Feuchtigkeitsabsorbierendes Tuch
Drap absorbant l'humidité

(30) Priority: 12.01.1993 NL 9300054
(43) Date of publication of application: 17.08.1994
(73) Proprietor: Havik, Marinus, Locate Varesino (IT)
(72) Inventor: Havik, Marinus, Locate Varesino (IT)
(74) Representative: Vollebregt, Cornelis Jacobus, Ir.

(56) References cited:
- EP-A- 0 110 287
- EP-A- 0 423 436
- DE-U- 9 112 275
- US-A- 4 499 131
- US-A- 4 772 281
- J.Daintith: A DICTIONARY OF PHYSICAL SCIENCES

## Description

The invention relates to a moisture-absorbing structure, in particular a pad, provided with at least one moisture-absorbing layer, which is provided on a layer which is impervious to liquid, as known from DE-U-9112275.

Such moisture-absorbing structures have been known for many years and are used in large quantities, for example as incontinence pads. A drawback of the existing structures is that generally they can be used only once, since they cannot be washed very well, whilst furthermore the impervious layer is made of a material which gives off harmful substances upon being incinerated.

According to the invention the impervious layer is made of a halogenated interpolymer with a base of ethene and a single top layer consisting of cotton and having a terry cloth structure is provided on top of said impervious layer. In practice it has appeared to be very well possible to combine an impervious layer made of such a material with a moisture-absorbing layer, whereby a structure of this type lends itself particularly well for repeated use, since such a structure can also be washed at high temperatures, washing water of up to ± 90 °C, without any unequal shrinkage among the layers, which may lead to undesirable deformation of the structure. The percentage of shrinkage of the pad appears to be virtually nil thereby. In addition to that the impervious layer does not contain any substances which are harmful for the environment.

It is noted that from US-A-4499131 it is known that e.g. neoprene allows for moisture resistibility in reasable moisture impervious underpads.

The invention will be explained in more detail hereafter with reference to two possible embodiments of the moisture-absorbing structure according to the invention diagrammatically illustrated in the accompanying Figure.

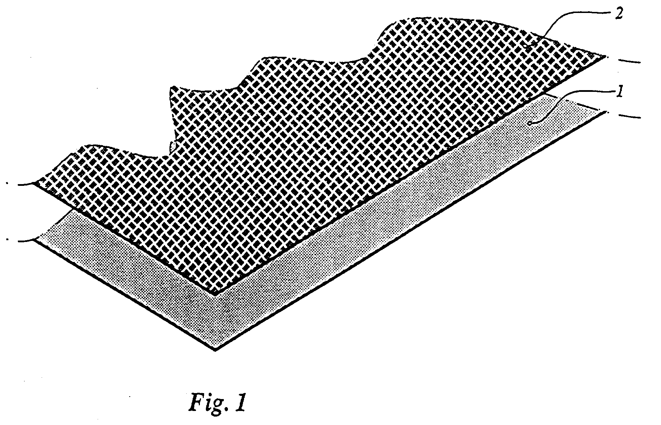

Figure 1 shows a structure according to the invention, consisting of two layers.

The pad shown in Figure 1 is built up of an impervious layer, which is made of a halogenated interpolymer with a base of ethene, more in particular of alcryn (trademark of Dupont).

A layer 2, which for easy reference is shown spaced from the impervious layer 1, is adhered to said layer 1, said layer 2 having a so-called terry cloth structure and consisting of a hundred per cent cotton.

The layer 2 was pressed on the layer 1 whilst the material of layer 1 was still slightly sticky, so as to make layer 2 adhere to layer 1 over the entire surface.

The construction is thereby such that 46 per cent by weight of the structure shown in Figure 1 consists of said halogenated interpolymer with a base of ethene, whilst 54 per cent by weight consists of cotton. More in particular the structure shown in Figure 1 contains 350 g/m² halogenated interpolymer with a base of ethene and 400 g/m² cotton. In practice it has appeared that the moisture absorption by the moisture-absorbing layer 2 amounts to 1.4 l/m² with this construction.

## Claims

1. A moisture-absorbing structure, in particular a pad, provided with at least one moisture-absorbing layer (2), which is provided on a layer (1), which is impervious to liquid, characterised in that said impervious layer (1) is made of a halogenated interpolymer with a base of ethene and a single top layer (2) consisting of cotton and having a terry cloth structure provided on top of said impervious layer (1).

2. A moisture-absorbing structure according to claim 1 or 2, characterised in that said structure (1, 2) contains 54 percent by weight cotton and 46 percent by weight halogenated interpolymer with a base of ethene.

3. A moisture-absorbing structure according to any preceding claim, characterised in that said cotton top layer (2) weights ± 400 g/m² and that said impervious layer (1) consisting of halogenated interpolymer with a base of ethene weights ± 340 g/m².

## Patentansprüche

1. Feuchtigkeitsaborbierende Struktur, insbesondere ein Polster, die mit mindestens einer feuchtigkeitsabsorbierenden Schicht (2) versehen ist, die auf einer für Flüssigkeit undurchlässigen Schicht (1) vorgesehen ist,
**dadurch gekennzeichnet, daß**
die undurchlässige Schicht (1) aus einem halogenhaltigen Mischpolymerisat auf Ethylenbasis und einer einzelnen Deckschicht (2) hergestellt ist, welche aus Baumwolle besteht und eine Schlingengewebestruktur aufweist, die auf der Oberseite der undurchlässigen Schicht (1) vorgesehen ist.

2. Feuchtigkeitsabsorbierende Struktur nach Anspruch 1,
**dadurch gekennzeichnet, daß**
die Struktur (1, 2) 54 Gewichtsprozent Baumwolle und 46 Gewichtsprozent halogenhaltiges Mischpolymerisat auf Ethylenbasis enthält.

3. Feuchtigkeitsabsorbierende Struktur nach einem der vorangehenden Ansprüche,
**dadurch gekennzeichnet, daß**
die Baumwolldeckschicht (2) ± 400 g/m² und die aus halogenhaltigem Mischpolymerisat auf Ethylenbasis bestehende undurchlässige Schicht (1) ± 340 g/m² wiegt.

## Revendications

1. Structure absorbant l'humidité en particulier un tampon, comprenant au moins une couche (2) absorbant l'humidité, laquelle est disposée sur une couche (1) imperméable aux liquides, caractérisée en ce que ladite couche imperméable (1) est constituée d'un interpolymère halogéné avec une base d'éthène et une couche supérieure (2) unique en coton à structure spongieuse disposée sur le dessus de ladite couche imperméable (1).

2. Structure absorbant l'humidité selon la revendication 1, caractérisée en ce que ladite structure (1, 2) contient 54 % en poids de coton et 46 % en poids d'un interpolymère halogéné à base d'éthène.

3. Structure absorbant l'humidité selon l'une quelconque des revendications précédentes, caractérisée en ce que ladite couche supérieure (2) en coton pèse ± 400 g/m² et en ce que ladite couche imperméable (1) en interpolymère halogéné à base d'éthène pèse ± 340 g/m².
